(19) Europäisches Patentamt
European Patent Office
Office européen des brevets

(11) Veröffentlichungsnummer : **0 107 834**
**B1**

# (12) EUROPÄISCHE PATENTSCHRIFT

(45) Veröffentlichungstag der Patentschrift : **30.07.86**

(51) Int. Cl.⁴ : **A 61 K 7/13**

(21) Anmeldenummer : **83110282.7**

(22) Anmeldetag : **15.10.83**

## (54) Haarfärbemittel.

(30) Priorität : **23.10.82 DE 3239295**

(43) Veröffentlichungstag der Anmeldung : **09.05.84 Patentblatt 84/19**

(45) Bekanntmachung des Hinweises auf die Patenterteilung : **30.07.86 Patentblatt 86/31**

(84) Benannte Vertragsstaaten : **AT BE CH DE FR GB IT LI NL SE**

(56) Entgegenhaltungen :
**EP-A- 0 043 436**
**BE-A- 660 519**
**DE-A- 453 277**
**FR-A- 2 258 167**
**CHEMISCHE BERICHTE, Band 86, Nr. 12, 1953, Seiten 1492-1494, Weinheim, DE., H. KÄMMERER et al.: "Zur Darstellung von Oxylmethylphenolen durch hydrierende Dehalogenierung kernhalogenierter Oxymethylphenole"**
**BULL. SOC. CHIM. FRANCE, 1947, Seiten 676-679, Paris, FR., L. PALFRAY et al.: "Hydrogénation de quelques éthers phénoliques par le nickel Raney"**
**JOURNAL OF THE AMERICAN CHEMICAL SOCIETY, Band 73, 1951, Seiten 43-45, Easton, P.A., USA, E.L. ELIEL: "The structure of the Guaiacol "Mannich Bases""**
**"Beilsteins Handbuch der organischen Chemie", 4. Auflage, Band 6, zweites Ergänzungswerk, 1944, Seite 1083, Springer Verlag, Berlin, DE.**
**CHEMICAL ABSTRACTS, Band 19, 1925, Seiten 976-977, Columbus, Ohio, USA, L. HELFER: "2-Hydroxy-5-methoxycyclohexylcarbinol"**
**Die Akte enthält technische Angaben, die nach dem Eingang der Anmeldung eingereicht wurden und die nicht in dieser Patentschrift enthalten sind.**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Rose, David, Dr.**
**Holbeinweg 7**
**D-4010 Hilden (DE)**
Erfinder : **Lieske, Edgar**
**Hunsrückenstrasse 40**
**D-4000 Düsseldorf (DE)**

EP 0 107 834 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve 18, rue St-Denis, 75001 Paris, France

**Beschreibung**

Gegenstand der Erfindung sind Haarfärbemittel auf der Basis von Oxidationsfarbstoffen. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt, die unter dem Einfluß von Oxidationsmitteln oder von Luftsauerstoff Farbstoffe ausbilden. Als kosmetische Träger für die Oxidationsfarbstoffvorproukte dienen Cremes, Emulsionen, Gele, Shampoos, Schaumaerosole oder andere Zubereitungen, die für die Anwendung auf dem Haar geeignet sind.

Für das Färben von Haaren spielen die sogenannten Oxidationsfarben, die durch oxidative Kupplung einer oder mehrerer Entwicklerkomponenten untereinander oder mit einer oder mehreren Kupplerkomponenten entstehen, wegen ihrer intensiven Farben und guten Echtheitseigenschaften eine bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopyridinderivate, heterocyclische Hydrazonderivate, 4-Aminopyrazolonderivate und Tetraaminopyrimidine eingesetzt. Als sogenannte Kupplersubstanzen werden m-Phenylendiaminderivate, Phenole, Naphthole, Resorcinderivate und Pyrazolone verwendet.

Gute Oxidationshaarfarbstoffvorprodukte müssen in erster Linie folgende Voraussetzungen erfüllen : Sie müssen bei der oxidativen Kupplung die gewünschten Farbnuancen in ausreichander Intensität ausbliden. Diese Farbnuancen müssen gegen die Einwirkung von Wärme, Licht und Chemikalien, z. B. gegen die bei der Kaltwellbehandlung des Haares verwendeten Reduktionsmittel eine ausreichende Stabilität aufweisen. Sie müssen ferner ein gutes Aufziehvermögen auf menschlichem Haar besitzen ohne die Kopfhaut zu stark anzufärben, und sie sollen vor allem in toxikologischer und dermatologischer Hinsicht unbedenklich sein.

Diese Anforderungen werden von den zur Zeit in Oxidationshaarfärbemitteln gebräuchlichen Kupplern, insbesondere von Kupplern, die mit bekannten Entwicklern wie p-Toluylendiamin blaue Nuancen ergeben, nicht zufriedenstellend erfüllt. Besonders problematisch sind die toxikologischen Eigenschaften vieler Kuppler z. B. vom Typ der aromatischen Amine. Andere Kupplersubstanzen führen zu Färbungen mit nicht befriedigenden Echtheitseigenschaften.

Aus der europäischen Patentanmeldung Nr. 43436 sind Haarfärbemittel bekannt, die substituierte Phenole mit jeweils einem Hydroxyalkylsubstituenten und gegebenenfalls einem Alkylsubstituenten mit je 1 bis 4 Kohlenstoffatomen als Kupplersubstanz enthalten. Auch diese Kuppler erfüllen noch nicht die an die Thermostabilität gestellten Anforderungen, denn die gefärbten und der Wärme ausgesetzten Haare neigen zu Farbveränderungen.

Aus der französichen Patentanmeldung FR-A-2 258 167 war die Verwendung von hydroxyalkylsubstituierten 1,2-Dihydroxy-benzolen als Haarfarbstoffvorprodukt bekannt. Diese Verbindungen weisen jedoch keine befriedigenden Kupplereigenschaften auf.

Bei der Suche nach besseren Oxidationsfarbstoffvorprodukten bestand daher die Aufgabe, geeignete Kupplersubstanzen aufzufinden, die sowohl in dermatologischer als auch in färberischer Hinsicht und bezüglich der Echtheitseigenschaften die gestellten Anforderungen in optimaler Weise erfüllen.

Diese Aufgabe wurde erfindungsgemäß gelöst durch neue Haarfärbemittel, enthaltend Oxidationsfarbstoffvorprodukte in einem kosmetischen Träger, dadurch gekennzeichnet, daß als Oxidationsfarbstoffvorprodukte substituierte Phenole der allgemeinem Formel I

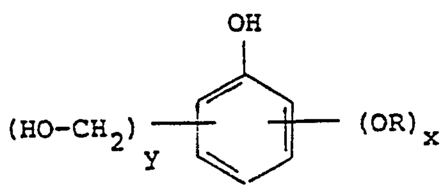

(I)

in der R eine Alkylgruppe mit 1 bis 4 Kohlenstoffatomen, x Werte von 0 bis 2 und y die Werte 1 oder 2 bedeuten und die Summe x + y 2 oder 3 beträgt, wobei die Substituenten —OR und —$CH_2OH$ beliebige Positionen einnehmen können, als Kupplerkomponenten und die in Oxidationsfärbemitteln üblichen Entwicklersubstanzen enthalten sind. Unter den Kupplersubstanzen der allgemeinen Formel I sind solche, bei denen eine Orthoposition oder die Paraposition zur phenolischen Hydroxylgruppe unsubstituierte ist, am besten geeignet.

Die Verbindungen der allgemeinen Formel I sind literaturbekannt. Einige der Verbindungen, z. B. Vanillylalkohol (2-Methoxy-4-hydroxymethyl-phenol) und Isovanillyl-alkohol (2-Methoxy-5-hydroxymethphenol) sind im Chemikalienhandel erhältlich.

Die erfindungsgemäßen Haarfärbemittel erzeugen auf dem Haar sehr intensive, tiefbaue bis rotbraune Farbnuancen von hoher Reinheit und mit guter Beständigkeit gegen Licht und Wärme. Sie stellen daher eine wesentliche Bereicherung der oxidativen Haarfärbemöglichkeiten dar.

Die neuen Kupplersubstanzen der allgemeinen Formel I eignen sich für eine Vielzahl verschiedener Entwicklersysteme. Besonders intensive und reine blaue Farbnuancen werden jedoch vor allem bei Verwendung von aromatischen Diaminen, besonders von 1.4-Diaminen und deren Derivaten als Entwicklersubstanzen erhalten. Solche bevorzugten Entwicklersysteme sind z. B. p-Phenylendiamin, p-Tolylendiamin, N-Methyl-p-phenylendiamin, N,N-Dimethyl-p-phenylendiamin, N-N-Diethyl-2-methyl-p-phenylendiamin, N-Ethyl-N-hydroxyethyl-p-phenylendiamin, Chlor-p-phenylendiamin, N,N-Bis-(2-hydroxyethyl)-p-phenylendiamin, Methoxy-p-phenylendiamin, 2,6-Dichlor-p-phenylendiamin, 2-Chlor-6-brom-p-phenylendiamin, 2-Chlor-6-methyl-p-phenylendiamin, 6-Methoxy-3-methyl-p-phenylmendiamin, 2,5-Diaminoanisol, N-Ethyl-(N-(2-hydroxyethyl)-p-phenylendiamin, N-2-Methoxyethyl-p-phenylendiamin, N-2-Hydroxypropyl-p-phenylendiamin, N-Butyl-N-sulfobutyl-p-phenylendiamin.

Daneben können die erfindungsgemäßen Haarfärbemittel jedoch zur Erzeugung der gewünschten Farbtöne noch andere bekannte Entwickler sowie Kupplerkomponenten enthalten. Solche Entwickler sind z. B. p-Aminophenole, Diaminopyridin-Derivate, heterocyclische Hydrazonderivate, 4-Aminopyrazolonderivate und Tetraaminopyrimidine. Als weitere Kuppler können den erfindungsgemäßen Haarfärbemitteln z. B. Naphthole, Resorcinderivate, Pyrazolone und m-Phenylendiaminderivate zugesetzt werden. Auch direktziehende Farbstoffe, z. B. Nitrophenylendiaminderivate können zur Modifikation der Farbnuancen zugesetzt werden.

In den erfindungsgemäßen Haarfärbemitteln werden die Kupplersubstanzen im allgemeinen in etwa molaren Mengen, bezogen auf die verwendeten Entwicklersubstanzen, eingesetzt. Wenn sich auch der molare Einsatz als zweckmäßig erweist, so ist es jedoch nicht nachteilig, einen gewissen Überschuß einzelner Oxidationsfarbstoffvorprodukte zum Einsatz zu bringen.

Es ist ferner nicht erforderlich, daß die Oxidationsfarbstoffvorprodukte einheitliche Substanzen darstellen, vielmehr können sowohl die Entwicklerkomponente Gemische der erfindungsgemäß zu verwendenden Entwicklerverbindungen als auch die Kupplerkomponente Gemische der erfindungsgemäß einzusetzenden substituierten Phenole der allgemeinen Formel I und bekannter Kupplersubstanzen darstellen.

Die oxidative Entwicklung der Färbung kann grundsätzlich, wie bei anderen Oxidationshaarfarbstoffen auch, durch Luftsauerstoff erfolgen. Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Zur Herstellung der erfindungungsgemäßen Haarfärbemittel werden die Oxidationsfarbstoff-Vorprodukte in einen geeigneten kosmetischen Träger eingearbeitet. Solche Träger sind z. B. Cremes, Emulsionen, Gele oder auch tensidhaltige, schäumende Lösungen, z. B. Shampoos oder andere Zubereitungen, die für die Anwendungen auf dem Haar geeignet sind. Übliche Bestandteile solcher kosmetischer Zubereitungen sind zum Beispiel Netz- und Emulgiermittel, wie anionische, nichtionische oder ampholytische Tenside, z. B. Fettalkoholsulfate, Alkansulfonate, α-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel wie z. B. Methyl- oder Hydroxyethylcellulose, Stärke, Fettalkohole, Paraffinöl, Fettsäuren, ferner Parfümöle und haarpflegende Zusätze wie z. B. wasserlösliche kationische Polymere, Proteinderivate, Pantothensäure und Cholesterin.

Die Bestandteile der kosmetischen Träger werden zur Herstellung der erfindungsgemäßen Haarfärbemittel in für diese Zwecke üblichen Mengen eingesetzt; z. B. werden Emulgiermittel in Konzentrationen von 0,5 bis 30 Gew.-% und Verdickungsmittel in Konzentrationen von 0,1 bis 25 Gew.-% der Haarfärbemittel eingesetzt. Die Oxidationsfarbstoffvorprodukte werden in Mengen von 0,05 bis 5,0 Gew.-%, vorzugsweise 1 bis 3 Gew.-% des gesamten Färbemittels in den Träger eingemischt.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8-10 erfolgen. Die Anwendungstemperaturen bewegen sich dabei im Bereich von 15 bis 40 °C. Nach einer Einwirkungsdauer von ca. 30 Minuten wird das Haarfärbemittel vom zu färbenden Haar durch Spülen entfernt. Hernach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Die mit den erfindungsgemäßen Haarfärbemitteln erzielbaren Färbungen haben gute Licht-, Wasch- und Reibechtheitseigenschaften.

Die nachfolgenden Beispiele sollen den Erfindungsgegenstand näher erläutern, ohne ihn jedoch hierauf zu beschränken.

## Beispiele

Zur Prüfung der erfindungsgemäßen Haarfärbemittel wurden die substituierten Phenole der allgemeinen Formel I des Patentanspruchs 1 als Kupplerkomponenten in Haarfärbe-Cremeemulsionen der folgenden Zusammensetzung eingesetzt:

| | |
|---|---|
| Fettalkohol $C_{12-18}$ | 10 g |
| Fettalkohol $C_{12-18}$-sulfat, Na-Salz | 10 g |
| Wasser | 75 g |
| Entwicklersubstanz | 0,007 5 Mol |
| Kupplersubstanz | 0,007 5 Mol |
| konz. Ammoniak-Lösung | bis pH = 9,5 |
| Wasser | ad 100 g |

Die Bestandteile wurden der Reihe nach miteinander vermischt. Nach Zugabe der Entwicklersubstanz und der Kupplersubstanz wurde zunächst mit konzentrierterAmmoniaklösung der pH-Wert der Emulsion auf 9,5 eingestellt, dann mit Wasser auf 100 g aufgefüllt.

Die oxidative Kupplung wurde mit 9 %iger Wasserstoffperoxidlösung als Oxidationsmittel durchgeführt. Hierzu wurden 100 g der Emulsion mit 10 g Wasserstoffperoxidlösung (9 %ig) versetzt und vermischt.

Die Färbecreme wurde auf standardisiertes, zu 90 % ergrautes aber nicht besonders vorbehandeltes Menschenhaar aufgetragen und dort 30 Minuten bei 35 °C belassen. Nach Beendigung des Färbeprozesses wurde das Haar gespült, mit einem üblichen Haarwaschmittel ausgewaschen und anschließend getrocknet.

Die als Entwicklersubstanzen verwendeten Stoffe waren

E 1 : p-Phenylendiamin
E 2 : p-Toluylendiamin
E 3 : N-Methyl-p-phenylendiamin
E 4 : 2-Chlor-p-phenylendiamin
E 5 : 2.5-Diaminoanisol
E 6 : N-Ethyl-N-(2-hydroxyethyl)-p-phenylendiamin
E 7 : N-Butyl-N-sulfobutyl-p-phenylendiamin
E 8 : N.N-Bis-(2-hydroxyethyl)-p-phenylendiamin
E 9 : N-(2-methoxyethyl)-p-phenylendiamin
E 10 : N-(2-hydroxypropyl)-p-phenylendiamin
E 11 : p-Aminophenol
E 12 : 2,4,5,6-Tetraaminopyrimidin
E 13 : 2-Dimethylamino-4.5.6-triaminopyrimidin.

Als Kupplersubstanzen wurden folgende Verbindungen verwendet :

K 1 : 2-Methoxy-5-hydroxymethyl-phenol
K 2 : 2-Methoxy-4-hydroxymethyl-phenol
K 3 : 2-Ethoxy-4-hydroxymethyl-phenol
K 4 : 2-Methoxy-6-hydroxymethyl-phenol
K 5 : 2-Hydroxymethyl-4-methoxy-phenol
K 6 : 2.5-Bis-(hydroxymethyl)-4-methoxy-phenol
K 7 : 2.6-Bis-(hydroxymethyl)-phenol
K 8 : 2.6-Bis-(methoxy)-4-hydroxymethyl-phenol

Die Herstellung der Verbindungen ist literaturbekannt :

K 1 : (Isovanillylalkohol) ist erhältlich durch EGA-Chemie, Steinheim
K 2 : (Vanillylalkohol) ist erhältlich durch EGA-Chemie, Steinheim
K 3 : wurde synthetisiert nach L. Palfray u. B. Gauthier, Bull. Soc. Chim. France, 1947, Seite 679
K 4 : wurde synthetisiert nach E. L. Eliel, J. Am. Chem. Soc. *73*, (1951), Seite 44 (o-Vanillylalkohol)
K 5 + K 6 : wurden synthetisiert nach L. Helfer, Helv. Chim. Acta *7* (1924), Seite 955
K 7 : wurde synthetisiert nach H. Kämmerer u. M. Grossmann, Chem. Ber. *86*, (1953), Seite 1492-1493
K 8 : wurde hergestellt nach DE-PS 453 277

Zum Vergleich wurden die folgenden bekannten Kuppler geprüft :

K 9 : 2-Methyl-4-hydroxymethyl-phenol
K 10 : 2-Hydroxymethyl-phenol
K 11 : 3-Hydroxymethyl-phenol

Die Ergebnisse der Färbeversuche sind der Tabelle I und II zu entnehmen.

Prüfung der Thermostabilität

Die angefärbten Haarsträhnen wurden in einem thermostatisierten Trockenschrank 7 Tage lang bei + 45 °C gelagert. Nach dieser Wärmelagerung wurde die Farbnuance mit der einer bei + 20 °C gelagerten Probe der gleichen Anfärbung verglichen. Die Ergebnisse sind der Tabelle II zu entnehmen.

Tabelle I

| Beispiel Nr.: | Entwickler | Kuppler | Nuance des gefärbten Haares |
|---|---|---|---|
| 1 | E 1 | K 1 | dunkelviolett |
| 2 | E 2 | K 1 | blauviolett |
| 3 | E 3 | K 1 | schwarzblau |
| 4 | E 4 | K 1 | dunkelviolett |
| 5 | E 5 | K 1 | tintenblau |
| 6 | E 6 | K 1 | dunkelbau |
| 7 | E 7 | K 1 | mattblau |
| 8 | E 8 | K 1 | dunkelblau |
| 9 | E 9 | K 1 | schwarzblau |
| 10 | E 10 | K 1 | schwarzblau |
| 11 | E 11 | K 1 | dunkelbraun |
| 12 | E 12 | K 1 | braunorange |
| 13 | E 13 | K 1 | rotblond |
| 14 | E 12 | K 2 | rotblond |
| 15 | E 12 | K 7 | hellbraun |

(Siehe Tabelle 2 Seite 6 f.)

Tabelle II

| Beispiel Nr. | Entwickler | Kuppler | Nuance des gefärbten Haares | Thermostabilität 7 Tage, 45°C |
|---|---|---|---|---|
| 2 | E 2 | K 1 | blauviolett | blauschwarz |
| 16 | E 2 | K 2 | blauviolett | unverändert |
| 17 | E 2 | K 3 | schwarz-blauviolett | unverändert |
| 18 | E 2 | K 4 | schwarzblau | unverändert |
| 19 | E 2 | K 5 | schwarzblau | unverändert |
| 20 | E 2 | K 6 | dunkelbauviolett | schwarzblau |
| 21 | E 2 | K 7 | schwarzblau | unverändert |
| 22 | E 2 | K 8 | dunkelviolett | unverändert |
| 23 | E 2 | K 9 | schwarzblau | leicht verbräunt |
| 24 | E 2 | K 10 | schwarzblau | schwarz, verbräunt |
| 25 | E 2 | K 11 | schwarzblau | braungrau |

**Patentansprüche**

1. Haarfärbemittel, enthaltend Oxidationsfarbstoffvorprodukte in einem kosmetischen Träger, dadurch gekennzeichnet, daß als Oxidationsfarbstoffvorprodukte substituierte Phenole der allgemeinen Formel

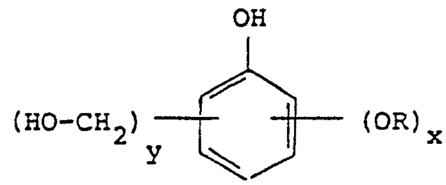

in der R eine Alkylgruppe mit 1 bis 4 Kohlenstoffatomen, x Werte von 0 bis 2 und y die Werte 1 oder 2 bedeuten und die Summe x + y 2 oder 3 beträgt, wobei die Substituenten —OR und —$CH_2OH$ beliebige Positionen einnehmen können, als Kupplerkomponenten und die in Oxidationsfärbemitteln üblichen Entwicklersubstanzen enthalten sind.

2. Haarfärbemittel nach Anspruch 1, dadurch gekennzeichnet, daß R eine Methyl- oder Ethylgruppe darstellt.

3. Haarfärbemittel nach Anspruch 1 und 2, dadurch gekennzeichnet, daß wenigstens eine der Positionen 2, 4 und 6 des Phenols unsubstituiert ist.

4. Haarfärbemittel nach Anspruch 1 bis 3, dadurch gekennzeichnet, daß zusätzlich weitere bekannte Kupplersubstanzen und/oder direktziehende Farbstoffe enthalten sind.

5. Haarfärbemittel nach Anspruch 1 bis 4, dadurch gekennzeichnet, daß der Gehalt an Oxidationsfarbstoffvorprodukten 0,05 bis 5,0 Gew.-% des gesamten Färbemittels ausmacht.

**Claims**

1. Hair dyes containing oxidation dye precursors in a cosmetic carrier, characterized in that they contain as oxidation dye precursors substituted phenols corresponding to the following general formula

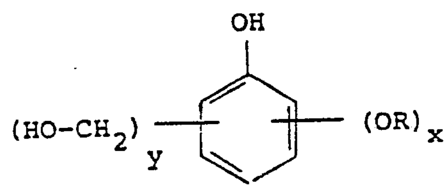

in which R is a $C_1$-$C_4$ alkyl group, x has a value of from 0 to 2 and y the value 1 or 2 and the sum of x + y is 2 or 3, the substituents —OR and —$CH_2OH$ occupying any positions, as coupler components and the developer substances normally used in oxidation hair dyes.

2. Hair dyes as claimed in Claim 1, characterized in that R is a methyl or ethyl group.

3. Hair dyes as claimed in Claims 1 and 2, characterized in that at least one of the 2, 4 and 6 positions of the phenol is unsubstituted.

4. Hair dyes as claimed in Claims 1 to 3, characterized in that they additionally contain other known coupler substances and/or substantive dyes.

5. Hair dyes as claimed in Claims 1 to 4, characterized in that the content of oxidation dye precursors makes up from 0.05 to 5.0 % by weight of the dye as a whole.

**Revendications**

1. Composition pour la teinture des cheveux contenant des précurseurs de colorants d'oxydation dans un support cosmétique, caractérisée en ce que des phénols substitués sont contenus comme précurseurs de colorants d'oxydation et répondent à la formule générale

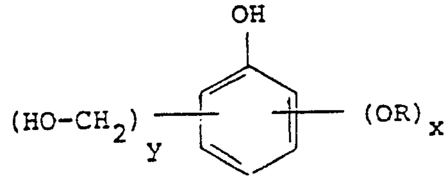

où R est un groupe alcoyl à 1 à 4 atomes de carbone, x des valeurs de 0 à 2, et y des valeurs de 1 ou 2, la somme x + y se montant à 2 ou 3, pendant que les substituants —OR et —$CH_2OH$ peuvent prendre des positions quelconques, et que l'on rencontre des substances de développement courantes dans les compositions de teinture d'oxydation, comme composant de copulation.

2. Composition pour la teinture des cheveux suivant la revendication 1, caractérisée en ce que R représente un groupe méthyl ou éthyl.

3. Composition pour la teinture des cheveux suivant les revendications 1 et 2, caractérisée en ce qu'au moins une des positions 2, 4 et 6 du phénol n'est pas substituée.

4. Composition pour la teinture des cheveux suivant les revendications 1 à 3, caractérisée en ce que, supplémentairement, d'autres substances copulantes connues et/ou des colorants directs y sont contenus.

5. Composition pour la teinture des cheveux suivant les revendications 1 à 4, caractérisée en ce que la teneur en précurseurs de colorants d'oxydation représente 0,05 à 5 % en poids de l'ensemble du produit pour la teinture.